# EUROPEAN PATENT SPECIFICATION

(11) **EP 1 306 056 B1**
(45) Date of publication and mention of the grant of the patent: **24.05.2006**
(21) Application number: 02450241.1
(22) Date of filing: 23.10.2002
(51) Int. Cl.: A61B 17/04

(54) **Tissue suturing instrument**
Chirurgisches Gerät zum Nähen von Gewebe
Appareil chirurgical pour réaliser des sutures de tissus

(30) Priority: 23.10.2001 US 330490 P; 23.01.2002 US 350034 P
(43) Date of publication of application: 02.05.2003
(73) Proprietor: Arthrex, Inc., Naples, Florida 34108 (US)
(72) Inventor: O'Quinn, Philip S., Naples, Florida 34116 (US); Weber, Robert M., Chino Hills, California 91709 (US); Schmieding, Reinhold, Naples, Florida 34108 (US)
(74) Representative: Kopecky, Helmut

(56) References cited:
- WO-A-91/06247
- WO-A-99/47050
- US-A- 1 822 330
- DATABASE WPI Section PQ, Week 197837 Derwent Publications Ltd., London, GB; Class P31, AN 1978-A1458A XP002224844 & SU 549 146 A (BLISKUNOV), 18 May 1977 (1977-05-18)

## Description

### BACKGROUND OF THE INVENTION

### 1. Field of the Invention:

The present invention relates to an instrument for surgical suturing, such as capsular suture plication, and arthroscopic repair of torn tissue, such as glenoid and meniscal repair.

### 2. Description of the Related Art:

Intracorporeal suturing, particularly suturing tissue during archroscopie surgery, presents challenges to a surgeon who must manipulate suturing instruments in confined spaces, and often through a relatively small incision. One device which has been developed for facilitating suturing during arthroscopic surgery is disclosed in U.S. Pat. No. 4,890,615. This instrument has a cannulated or hollow needle secured to a jaw positioned near the end of a long tube. The jaw can be manipulated by a hand grip to press the needle through the tissue to be sutured. Suture material is then fed through the tube from a spool attached to the hand grip into the hollow needle and therefore through the tissue. Enough suture material is advanced through the needle so that when the needle is withdrawn from the tissue and the instrument is removed from the incision, a portion of the suture material remains within the tissue. The suture material is then tied in a manner well-known in the art so as to secure the suture material to the tissue.

While the device disclosed by U.S. Pat. No. 4,890,615 generally is effective in suturing tissue, there are several disadvantages associated with using this instrument. For example, the instrument requires manually advancing the suture material through the needle by manipulating the spool, which is often somewhat difficult to do during surgery. In addition, the surgeon must verify that enough suture material has been advanced through the needle so that when the instrument is withdrawn from the incision, the suture material is not inadvertently pulled through the tissue. Furthermore, because the suture material must be fed up and through a hollow needle, tissue or other debris present in the surgical site may block the opening in the needle, making it difficult to pass the suture material through the needle. In addition, because rotating the spool against the braided suture material tended to cause the braided suture material to expand such that it was unable to pass through the tube, only monofilament suture material can generally be used. This is disadvantageous because braided suture material is generally stronger than monofilatnent suture material. Finally, this particular instrument cannot generally be used to simultaneously pass several segments of suture material through the tissue, which is required for certain types of sutures such as a mattress suture.
The surgical instrument disclosed in the WO 91/06247 was developed for use.in open and closed surgeries for threading a loop of suture material through a piece of tissue or wrapping a piece of suture material around a soft tissue structure. WO 91/06247 discloses for this purpose a prong on which suture is supported across an opening. This document teaches that the suture is supported on the distal face of the prong (the face of the prong that faces away from the needle)

The Caspari suture punch, disclosed in U.S. Pat. No. 5,522,820, was developed to provide a suturing instrument for arthroscopic surgery without the above-noted disadvantages. The Caspari suture punch is a hand instrument with a pivotable jaw and a Stationary jaw. The stationary jaw is provided with a needle that extends upwardly and has a hook on the proximal side. With the jaws closed, suture is threaded through an aperture in the movable jaw, such that the suture is disposed on the distal side of the needle of the lower jaw and is not engaged by the hook. The instrument is inserted through a relatively small incision in the patient with the jaws closed. With the jaws positioned adjacent the tissue to be sutured, the surgeon opens the jaws (by manipulating the finger grip of the instrument disposed outside the patient), and then closes the jaws to engage the tissue to be sutured. As this occurs, the needle penetrates the tissue to be sutured, and the suture is captured in the hook of the needle. When the movable jaw is then opened, the suture, which is captured in the hook, is drawn through the tissue. The instrument is then removed from the incision and the suture is secured around the tissue with a knot.

While the Caspari punch avoids some of the disadvantages of the prior spool-fed suture punch, it is directed to applications in which the tissue to be sutured must be grasped, and thus requires a movable jaw mechanism. It would be desirable to provide instruments for suturing which do not require movable jaw mechanisms and are suitable for applications such as rotator cuff repair, surgical plication of a capsule, i.e., capsulorrhaphy, and repair of meniscal tears.

A meniscal tear is a typical injury to the knee which can occur, for example, when the meniscus is displaced and caught between the femoral and tibial condyles during a sudden change of movement of the knee involving a combined flexion-rotation or extension-rotation motion. Meniscal tears were originally treated by removing the meniscus in an operation called a meniscectomy. However, results showed that removing the meniscus, either entirely or even partially, resulted in degenerative arthritis and instability in the knee.

As a result of the above-described complications, surgeons began treating meniscus tears with suturing techniques to retain as much of the meniscus as possible. However, suturing of a meniscal tear, like a meniscectomy, was originally an open technique, requiring a large incision and consequently longer periods of rehabilitation and recovery. Advances in instrumentation ultimately led to arthroscopic meniscal repair using long needles for passing suture through the tear

More recently, various tacks, screws and implants have been developed for meniscal repair, which can be used arthroscopically and simplify the surgery by eliminating the need for suturing altogether. For example, U.S. Pat. No. 6,056,778, assigned to the assignee of the present application, discloses a meniscal repair device provided with a plurality of opposed crescent-shaped grooves which can be used to mend meniscal tears.

Some surgeons, however, prefer to repair meniscal tears arthroscopically using suture, but seek to avoid the difficulties associated with using long needles. Accordingly, it would be desirable to provide an instrument for repairing torn meniscal tissue which facilitates the passing of suture through a torn meniscus arthroscopically.

### SUMMARY OF THE INVENTION

The present invention provides instrumentation for suturing tissue arthroscopically, in particular, hand instruments for repairing torn tissue. The instruments are used to pass suture through soft tissue, such as a torn meniscus or glenoid, or a shoulder capsule undergoing plication.

The instruments feature a needle slidably disposed in a tubular shaft. The needle is advanced from the shaft to pierce through the tissue to be repaired, With continued advancement, a hook disposed at the end of the needle engages a length of suture supported ahead of the advancing needle on a prong that extends from the end of the instrument shaft. The needle then is withdrawn, drawing a captured loop of the suture back through the pierced tissue. The suture loop is available for subsequent suturing or knot tying.

More specifically, tissue suturing begins by presenting the selected instrument, loaded with suture, through an arthroscopic cannula, for example. The operative end of the instrument is positioned, with the needle withdrawn, proximate the tissue to be repaired. The needle then is advanced to pierce the tissue. Further advancement of the needle brings it clear of the pierced tissue to approach a length of suture supported on the extended prong of the instrument. A hook formed toward the tip of the needle captures the suture. Drawing the needle back pulls a loop of the captured suture through the tissue.

Operation of the instrument is facilitated by additional features of the invention. One handed manipulation of the instrument, for example, is simplified by a thumb slide provided on the handle. The thumb slide operates to advance the needle through the tubular shaft to pierce tissue and capture the suture, and withdraw the needle to pull captured suture back through the tissue. In addition, the needle can be configured to be withdrawn completely from the instrument to facilitate suture manipulation and withdrawal of the instrument from the patient. Further, the instrument can be made to be disposable, and can be manufactured with a malleable shaft which can be bent into various configurations to facilitate access to tissue.

Other features and advantages of the present invention will become apparent from the following description of the invention which refers to the accompanying drawings.

### BRIEF DESCRIPTION OF THE DRAWINGS

Fig. 1 is a perspective view of a tissue suturing instrument according to a preferred embodiment of the present invention

Fig. 2 is a detailed perspective view of the operative end of the instrument of Fig. 1 being applied in a surgical step of torn tissue repair according to the present invention,

Fig. 3 is a detailed view of the operative end of the instrument of Fig. 1 being used in a further surgical step of torn tissue repair according to the present invention.

Fig. 4 is a perspective view of a tissue suturing instrument according to an alternative embodiment of the present invention.

Fig. 5 is a perspective detail view of the operative end of the tissue suturing instrument of Fig. 4.

Fig. 6 is a perspective view of the instrument of Fig. 4, shown with the needle in an advanced position.

Fig. 7 is an enlarged view of the operative end of the instrument as shown in Fig. 6.

Fig. 8 is a perspective view of the instrument of Fig. 4 shown loaded with a length of braided suture.

Fig. 9 is an enlarged view of the operative end of the instrument as shown in Fig. 8.

Fig. 10 illustrates a step of performing surgical suture plication according to the present invention.

Fig. 11 illustrates a further step of performing surgical suture plication according to the present invention.

Fig. 12 illustrates an additional step of performing surgical suture plication according to the present invention.

### DETAILED DESCRIPTION OF THE PREFERRED EMBODIMENTS

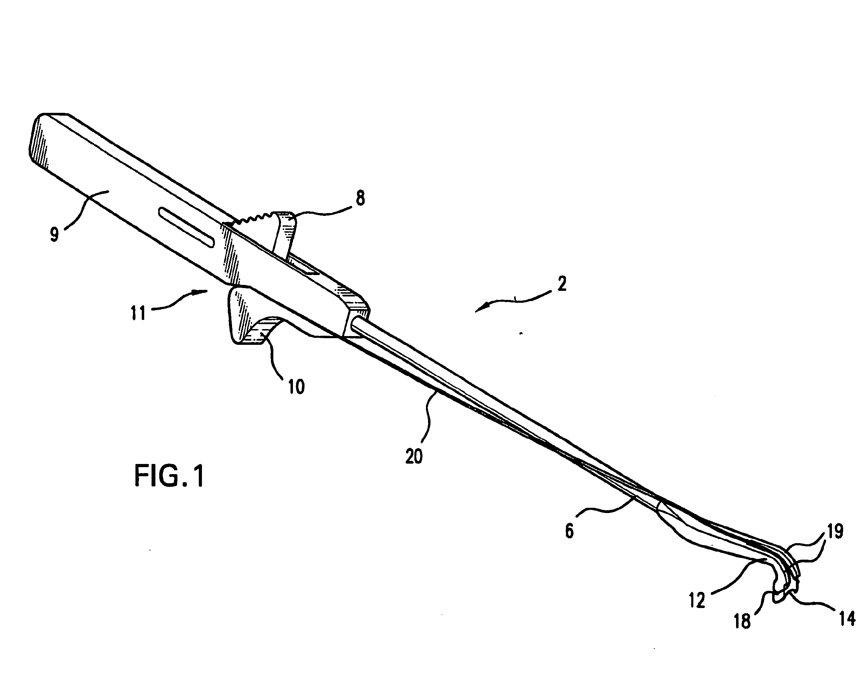

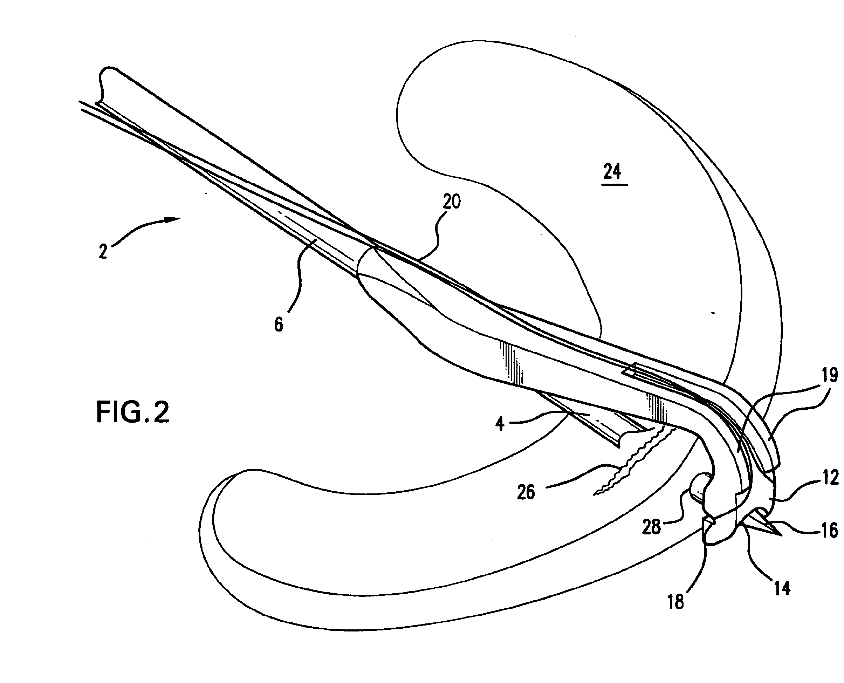

Referring initially to Fig. 1, a tissue suturing device 2 according to a preferred embodiment of the present invention includes a needle 4 (Fig. 2) that slides inside of a tubular shaft 6. Optionally, at least a portion of shaft 6 can be bendable, formed of a malleable material, for example, to facilitate directional control of suture placement. The needle is operated by a thumb slide 8 disposed on handle 9. A finger support 10 is provided for ease of handling. A suture notch 11 is used to secure loose ends of suture 20

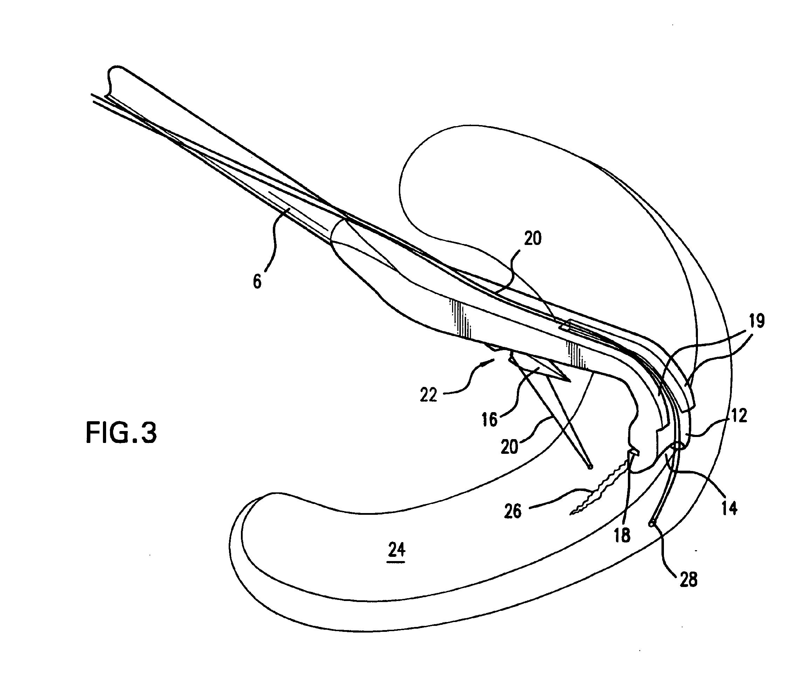

The working end of tube 6 is provided with an extended prong 12, shown in greater detail in Figs. 2 and 3. Prong 12 takes a flattened form that projects over and in advance of an extended end of the needle 4. An opening 14 provides a clearance in the distal end of prong 12. Manipulation of the instrument 2 with the thumb of one hand advances the needle 4. The opening 14 is aligned to provide passage for a tip portion of needle 4 when the needle is advanced.

A point 16 of the needle projects upon advancement through opening 14 to engage a length of suture, as follows: A slot 18 formed in the prong 12 holds braided suture 20. Referring also to Fig. 3, slot 18 intersects opening 14. Suture 20 passes up through a channel between ridges 19 and is held under tension using suture notch 11. Accordingly, a portion of suture 20 held in slot 18 transects opening 14. A hook 22 formed in the end of the needle 4 captures the portion of suture 20 when the needle is advanced through the opening.

More specifically, referring to Fig. 2, instrument 2 is brought by way of arthroscopic or open surgical means to tissue, such as meniscus 24, which is in need of repair due to a tear 26, for example. Braided suture 20 preferably is preloaded into slot 18. Needle 4 is advanced through the meniscal tissue and across the tear, piercing the meniscus 24 to form exit hole 28.

As the tip 16 of needle 4 passes through opening 14, hook 22 formed in the end of the needle captures the suture, and a loop of suture is drawn back through meniscus 24 as the needle is withdrawn by way of thumb slide 8, assisted by the retrograde urging of an optional spring. The loop of suture accordingly is available for further suturing and knot tying.

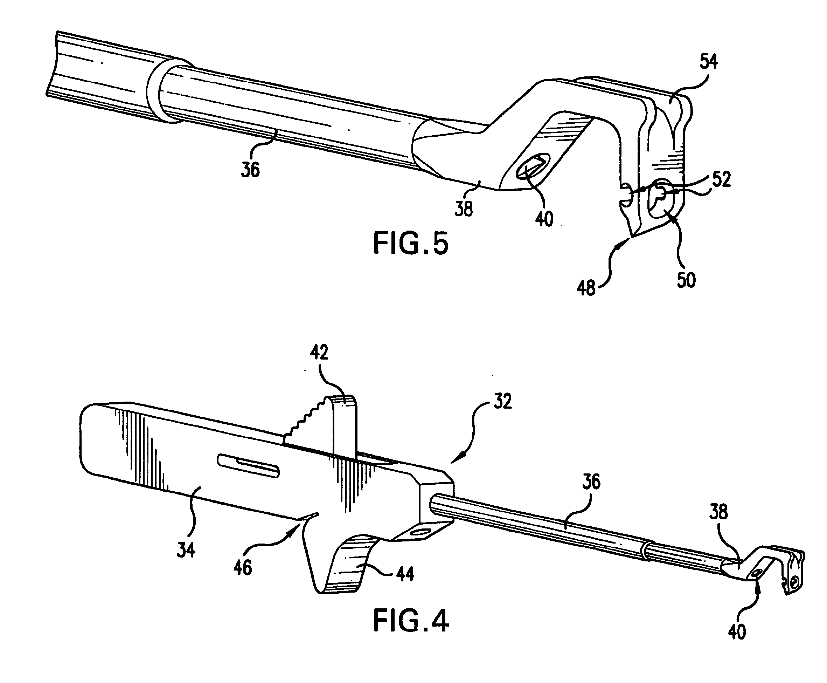

Referring to Figs. 4-12, a suturing instrument 32 according to an alternative embodiment of the present invention is shown. The instrument includes a handle 34 supporting a cannulated shaft 36. The cannulated shaft 36 terminates in a stationary prong 38. A needle 40 extends through shaft 36 and is operated by thumb slide 42. Needle 40 is shown in a retracted position in Fig. 4. Instrument 32 includes a finger support 44 for ease of handling, and a suture pinch slot 46 for capturing tensioned suture, as described below in connection with a preferred surgical procedure using the instrument.

Prong 38, illustrated in greater detail in Fig. 5, ends in a point 48. Above point 48, prong 38 is configured to define an opening 50 in axial alignment with needle 40. Slots 52 formed in prong 38 on either side of opening 50 are used to capture a section of a suture loop guided through channel 54 formed on the top of prong 38, as described further below.

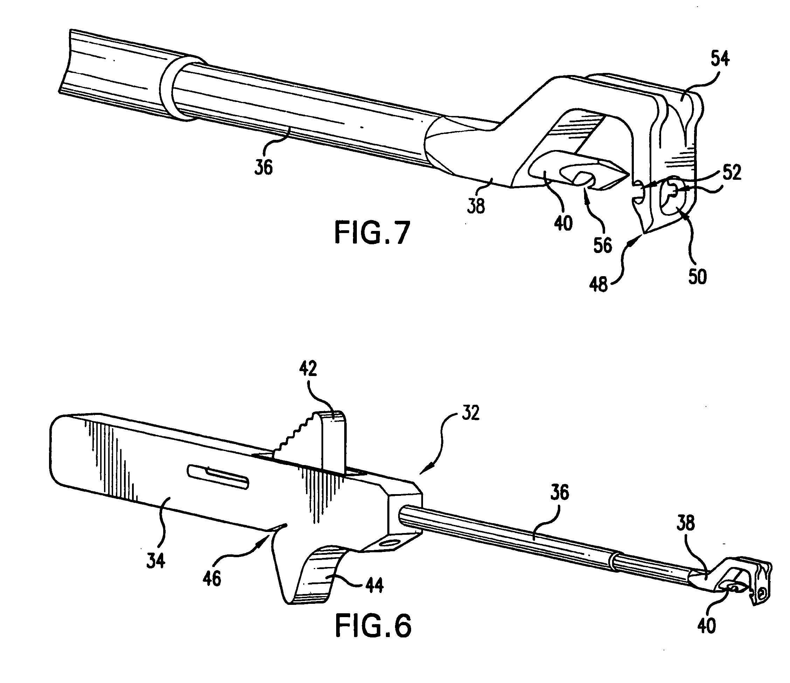

Referring to Figs. 6 and 7, instrument 32 is shown with needle 40 in a partially advanced position to reveal hook 56 formed at the end of needle 40. Hook 56 is shaped to capture suture held in slots 52 across opening 50 when needle 40 is advanced into opening 50, as described further below in connection with a preferred method of surgical suturing according to the present invention.

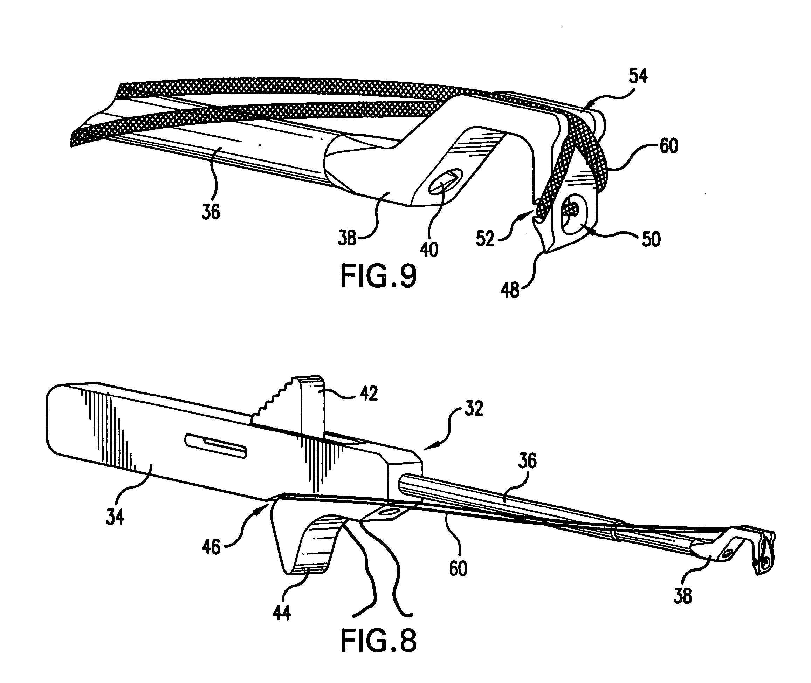

Referring to Figs. 8 and 9, instrument 32 is shown loaded with a length of braided suture 60 in preparation for performing surgical suturing. Suture 60 is looped through slots 52 across opening 50. The slots 52 face proximally toward needle 40. Two legs of the suture loop are passed upwardly and distally, and then proximally through channel 54 on top of prong 38. Suture 60 is passed along one side of instrument 32 and secured under tension in suture pinch slot 46.

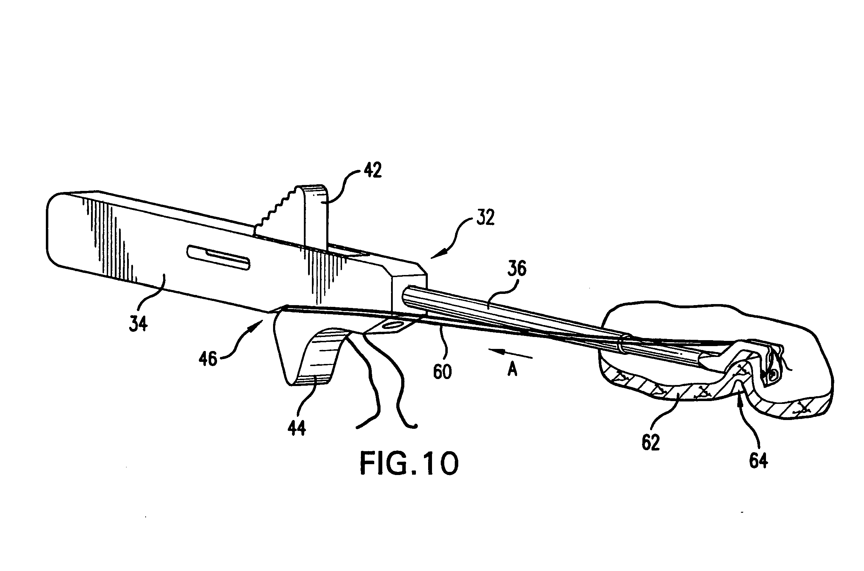

Referring to Fig. 10, a step is illustrated in a preferred procedure for performing surgical plication of a capsule 62. The capsulorrhaphy procedure is indicated for capsule reduction by securing folded pleats of tissue and thereby reduce laxity in the capsular tissue.

Instrument 32 is loaded with suture 60 as described above, and is inserted inferiorly along the capsule. Needle 40 of instrument 32 is maintained in a withdrawn position by manipulation of thumb slide 42.

Prong 38 of instrument 32 is shown surrounding a tuck or fold 64 of capsular tissue. The fold is formed by engaging the capsular tissue with pointed end 48 of instrument tip 38 and drawing the instrument back in the direction of arrow A in Pig. 10, The retrograde motion folds a significant amount of tissue into the prong.

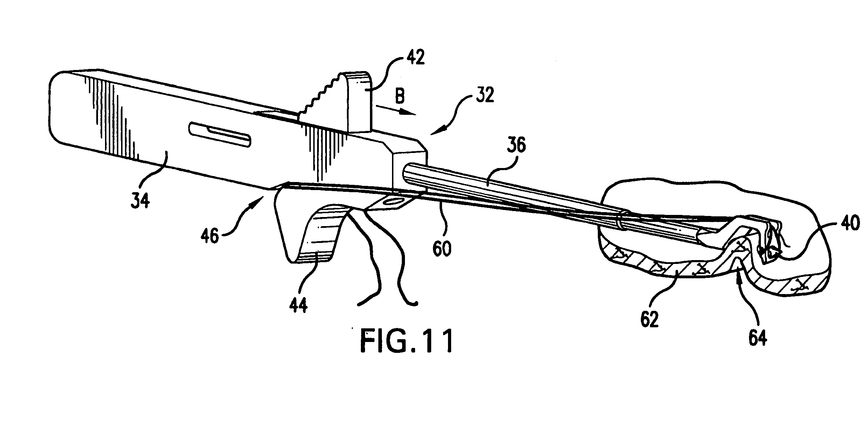

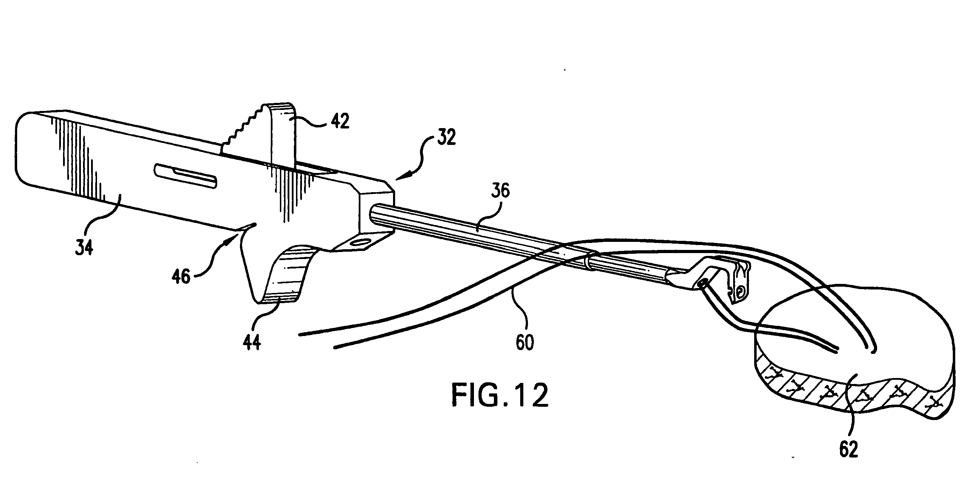

Referring to Fig. 11, the preferred capsular plication method proceeds with advancement of needle 40 through the tissue of fold 64. Needle 40 is advanced by the surgeon manipulating thumb slide 42 in the direction of arrow B of Fig. 12. As the end of needle 40 advances through tissue fold 64 and into opening 50, needle 40 engages the portion of suture 60 held across opening 50 between slots 52. Further advancement of needle 40 causes the portion of suture 60 to become captured by hook 56 (Fig. 8).

Referring to Fig. 12, instrument 32 is shown having been withdrawn from capsule 62, leaving suture 60 threaded through capsular tissue in preparation for suture knot tying to secure tightening fold 64. Prior to withdrawing the instrument 32, suture 60 is released from pinch slot 46, and needle 40 is withdrawn from the tissue fold by moving thumb slide 42 back. Suture 60 is then released from hook 56 and tied off to tighten the capsule, or one suture end can be inserted through the eyelet of a suture anchor prior to insertion as a combined capsular plication and Bankart repair.

Although the present invention has been described in connection with meniscal and glenoid repair, and capsular plication, the instrument also is useful for other surgical suturing procedures, particularly those involving endoscopic suturing of tissue.

While preferred embodiments of the invention has been described and illustrated above, it should be understood that these are exemplary of the invention and are not to be considered as limiting. Additions, deletions, substitutions, and other modifications can be made without departing from the scope of the present invention . Accordingly the invention is not to be considered as limited by the foregoing description but is only limited by the scope of the appended claims.

## Claims

1. An instrument (2) for surgical suturing, comprising:
a shaft (6);
a needle (4) disposed slidably for longitudinal travel with respect to the shaft (6);
a prong (12) on one end of the shaft (6), the prong (12) projecting across a longitudinal axis of the needle (4) and having an opening (14) formed through the prong (12) that provides a clearance through which a tip portion of the needle passes when it is advanced; and
slots (18) formed on a side of the prong (12) facing the end of the shaft (6) and supporting a length of suture (20) on an inner surface of the prong (12) facing the end of the shaft (6) such that the suture (20) extends across the opening formed through the prong (12), and a longitudinal suture channel formed on a top surface of the prong (12), the suture channel extending above the longitudinal axis of the needle (4), the suture (20) being held in the slots by passing strands of the suture (20) separately around sides of the prong (12) and together through the suture channel formed on the top surface of the prong.

2. The instrument of claim 1, further comprising a handle (9), wherein the shaft (6) is fixed nonslidably to the handle (9).

3. The instrument of claim 1, further comprising suture (20) supported in the slots (18) across the opening formed through the prong (12).

4. The instrument of one of the claims 1 to 3, for soft tissue suturing, **characterized in that**:
the tubular shaft(6) has proximal and distal ends;
the needle (4) is disposed slidably within the tubular shaft (6); and that
the prong (12) is formed on the distal end of the tubular shaft (6) for holding a length of suture(20) in a sliding path of the needle (4) such that a hook (22) formed on the needle captures the length of suture (20) and, upon proximal movement of the needle (4), the suture (20) is released from the prong (12), the suture being held in slots (18) formed on a proximal side of the prong (12) facing the end of the tubular shaft (6) by passing strands of the suture (20) separately around sides of die prong (12) and together through a suture channel formed on a top surface of the prong (12), the suture channel extending above a longitudinal axis of the needle (4).

## Patentansprüche

1. Instrument (2) zum chirurgischen Nähen, umfassend:
einen Schaft (6);
eine Nadel (4), die für eine Längsbewegung in Bezug auf den Schaft (6) verschiebbar angeordnet ist;
eine Zinke (12) an einem Ende des Schafts (6), wobei die Zinke (12) über eine Längsachse der Nadel (4) hinausragt und eine durch die Zinke (12) gebildete Öffnung (14) aufweist, die einen Zwischenraum schafft, durch den ein Spitzenteil der Nadel hindurchgeht, wenn diese vorgeschoben wird; und
Schlitze (18), die an einer dem Ende des Schafts (6) zugewandten Seite der Zinke (12) gebildet sind und an einer dem Ende des Schafts (6) zugewandten Innenfläche der Zinke (12) einen Abschnitt von Nahtmaterial (20) solcherart tragen, dass sich das Nahtmaterial (20) über die durch die Zinke (12) gebildete Öffnung erstreckt, sowie einen an einer Oberseite der Zinke (12) gebildeten, längsgerichteten Nahtkanal, wobei sich der Nahtkanal über der Längsachse der Nadel (4) erstreckt und das Nahtmaterial (20) in den Schlitzen gehalten wird, indem Stränge des Nahtmaterials (20) getrennt um die Seiten der Zinke (12) und zusammen durch den an der Oberseite der Zinke gebildeten Nahtkanal geführt werden.

2. Instrument nach Anspruch 1, weiters umfassend einen Griff (9), wobei der Schaft (6) in nicht verschiebbarer Weise am Griff (9) befestigt ist.

3. Instrument nach Anspruch 1, weiters umfassend ein in den Schlitzen (18) gehaltenes Nahtmaterial (20) quer über die durch die Zinke (12) gebildete Öffnung.

4. Instrument nach einem der Ansprüche 1 bis 3 für das Nähen von weichem Gewebe, **dadurch gekennzeichnet, dass**:
der röhrenförmige Schaft (6) ein proximales und ein distales Ende besitzt;
die Nadel (4) innerhalb des röhrenförmigen Schafts (6) verschiebbar angeordnet ist; und dass
die Zinke (12) am distalen Ende des röhrenförmigen Schafts (6) gebildet ist, um einen Abschnitt von Nahtmaterial (20) solcherart in einem Gleitpfad der Nadel (4) zu halten, dass ein an der Nadel gebildeter Haken (22) den Nahtmaterialabschnitt (20) erfasst und das Nahtmaterial (20) bei einer proximalen Bewegung der Nadel (4) von der Zinke (12) freigegeben wird, wobei das Nahtmaterial in Schlitzen (18) gehalten wird, die an einer dem Ende des röhrenförmigen Schafts (6) zugewandten, proximalen Seite der Zinke (12) gebildet sind, indem Stränge des Nahtmaterials (20) getrennt um die Seiten der Zinke (12) und zusammen durch einen an einer Oberseite der Zinke (12) gebildeten Nahtkanal geführt werden, wobei sich der Nahtkanal über einer Längsachse der Nadel (4) erstreckt.

## Revendications

1. Instrument (2) permettant de réaliser des sutures chirurgicales, comprenant :
une tige (6) ;
une aiguille (4) disposée de manière coulissante pour le déplacement longitudinal par rapport à la tige (6) ;
un fourchon (12) sur une extrémité de la tige (6), le fourchon (12) faisant saillie sur un axe longitudinal de l'aiguille (4) et ayant une ouverture (14) formée à travers le fourchon (12) qui propose un jeu à travers lequel une partie de pointe de l'aiguille passe lorsqu'elle avance ; et
des fentes (18) formées sur un côté du fourchon (12) faisant face à l'extrémité de la tige (6) et supportant une longueur de suture (20) sur une surface interne du fourchon (12) faisant face à l'extrémité de la tige (6) de sorte que la suture (20) s'étend sur l'ouverture formée à travers le fourchon (12), et un canal de suture longitudinal formé sur une surface supérieure du fourchon (12), le canal de suture s'étendant au dessus de l'axe longitudinal de l'aiguille (4), la suture (20) étant maintenue dans les fentes en faisant passer les brins de la suture (20) séparément autour des côtés du fourchon (12) et conjointement par le canal de suture formé sur la surface supérieure du fourchon.

2. Instrument selon la revendication 1, comprenant en outre une poignée (9), dans lequel la tige (6) est fixée de manière non coulissante sur la poignée (9).

3. Instrument selon la revendication 1, comprenant en outre la suture (20) supportée dans les fentes (18) sur l'ouverture formée à travers le fourchon (12).

4. Instrument selon l'une des revendications 1 à 3, permettant de suturer le tissu mou, **caractérisé en ce que** :
la tige (6) tubulaire a des extrémités proximale et distale ;
l'aiguille (4) est disposée de manière coulissante dans la tige (6) tubulaire ; et **en ce que**
le fourchon (12) est formé sur l'extrémité distale de la tige (6) tubulaire pour maintenir une longueur de suture (20) dans un trajet coulissant de l'aiguille (4) de sorte qu'un crochet (22) formé sur l'aiguille, capture la longueur de suture (20) et suite au mouvement proximal de l'aiguille (4), la suture (20) est libérée du fourchon (12), la suture étant maintenue dans les fentes (18) formées sur un côté proximal du fourchon (12) faisant face à l'extrémité de la tige (6) tubulaire en faisant passer des brins de la suture (20) séparément autour des côtés du fourchon (12) et conjointement à travers un canal de suture formé sur une surface supérieure du fourchon (12), le canal de suture s'étendant au dessus d'un axe longitudinal de l'aiguille (4).
